(19) Europäisches Patentamt
European Patent Office
Office européen des brevets

(11) Veröffentlichungsnummer: **0 265 812**
**B1**

(12) # EUROPÄISCHE PATENTSCHRIFT

(45) Veröffentlichungstag der Patentschrift: **08.08.90**

(51) Int. Cl.⁵: **A61K 31/675**, A61K 47/00, A61K 9/14

(21) Anmeldenummer: **87115322.7**

(22) Anmeldetag: **20.10.87**

(54) **Ifosfamid-Lyophilisat und Verfahren zu dessen Herstellung.**

(30) Priorität: **31.10.86 DE 3637089**

(43) Veröffentlichungstag der Anmeldung:
**04.05.88 Patentblatt 88/18**

(45) Bekanntmachung des Hinweises auf die Patenterteilung:
**08.08.90 Patentblatt 90/32**

(84) Benannte Vertragsstaaten:
**AT BE CH DE ES FR GB GR IT LI LU NL SE**

(56) Entgegenhaltungen:
**EP-A- 0 083 439**
**DE-A- 2 750 207**
**US-A- 3 732 340**
**US-A- 4 537 883**

**Die Akte enthält technische Angaben, die nach dem Eingang der Anmeldung eingereicht wurden und die nicht in dieser Patentschrift enthalten sind.**

(73) Patentinhaber: **ASTA Pharma Aktiengesellschaft, Weismüllerstrasse 45, D-6000 Frankfurt am Main 1(DE)**

(72) Erfinder: **Sauerbier, Dieter, Rauhe Horst 18, D-4806 Werther(DE)**
Erfinder: **Dammann, Uwe-Peter, Am Rott 35, D-4930 Detmold(DE)**
Erfinder: **Isaac, Otto, Dr., Liesingstrasse 8, D-6450 Hanau 9(DE)**

**EP 0 265 812 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Der chemische Name für den Wirkstoff Ifosfamid ist 3-(2-Chlorethyl)-2-(chlorethylamino)-tetrahydro-2H-1,3,2-oxazaphosphorin-2-oxid

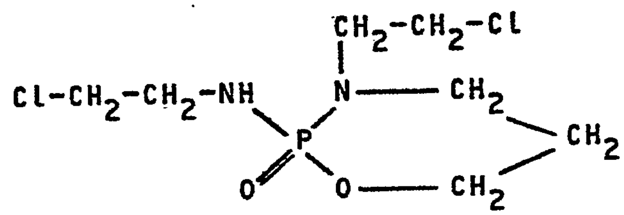

Ifosfamid gehört wie Cyclophosphamid zur chemischen Gruppe der Oxazaphosphorine und wird therapeutisch zur Behandlung von Tumor-Erkrankungen eingesetzt.

Ifosfamid ist ein weißes, kristallines Pulver mit einem Schmelzpunkt von 48°C bis 51°C und stark hygroskopischen Eigenschaften. Bereits unterhalb des Schmelzpunktes beginnt Ifosfamid zu sintern; es muß deshalb bei möglichst niedrigen Temperaturen (Raumtemperatur und darunter) gelagert werden. Außerdem ist ein Kontakt mit Luftfeuchtigkeit möglichst zu vermeiden.

Ifosfamid löst sich zu etwa 10 Gewichtsprozent in Wasser, ist aber in wäßriger Lösung nur begrenzt haltbar (maximal 3 bis 4 Stunden bei 20°C bis 22°C beziehungsweise 36 Stunden bei 4 bis 6°C).

Ifosfamid wird ausschließlich parenteral appliziert. Die Injektionsflaschen enthalten 200 bis 5000 mg Ifosfamid in Form eines Sterilkristallisats, das vor der Applikation in Wasser für Injektionszwecke gelöst wird, so daß eine 4%ige Konzentration nicht überschritten wird. Diese Lösung ist zur intravenösen Injektion geeignet. Zur intravenösen Kurzinfusion wird die Ifosfamid-Lösung in 500 ml Ringer-Lösung oder ähnlichen Infusionsflüssigkeiten aufgelöst. Die Infusionsdauer beträgt ca. 30 Minuten, eventuell 1 bis 2 Stunden. Bei der 24-Stunden-Infusion wird die Ifosfamid-Lösung beispielsweise in insgesamt 3 Liter 5% Dextrose-Kochsalzlösung aufgelöst.

Ifosfamid verursacht bei der Herstellung und Verarbeitung mannigfaltige Probleme. Bei der Herstellung des steril kristallisierten Ifosfamids resultiert ein Produkt von wechselnder physikalischer Beschaffenheit. Durch die unterschiedliche Rieselfähigkeit wird insbesondere die Dosierungsgenauigkeit bei der Abfüllung in hohem Maße beeinträchtigt.

Die Verarbeitung des Ifosfamids wird weiterhin erschwert durch seine Hygroskopizität und den niedrigen Schmelzpunkt. Bei längerer Lagerung sintert das Sterilkristallisat und die Lösungsgeschwindigkeit vermindert sich. Mit beginnender Sinterung des Ifosfamids nehmen auch die Klarlöslichkeit und der pH-Wert der Lösung bei gleichzeitiger Gelbfärbung ab; eine therapeutische Verwendung ist dann im allgemeinen nicht mehr möglich.

Aufgabe der Erfindung ist daher, Ifosfamid in einer Form mit verbesserten Eigenschaften, wie verbesserte Stabilität, Lagerfähigkeit, Dosierbarkeit und Löslichkeit, bereitzustellen, die leichter anzuwenden ist, und insbesondere zur Herstellung von injizierbaren Lösungen geeignet ist.

Es wurde nun überraschend gefunden, daß die bisherigen Nachteile und Schwierigkeiten bei der Handhabung und Lagerung von Ifosfamid durch Verwendung eines bestimmten Ifosfamid-Lyophilisats behoben werden können. Insbesondere ist es überraschend, daß das erfindungsgemäße Ifosfamid-Lyophilisat eine größere Thermostabilität besitzt als die bislang verwendete Ifosfamid-Trockenabfüllung.

Trockenabfüllungen mit Ifosfamid sind bei 40°C bereits nach einer Lagerzeit von 1 Monat nachgedunkelt; nach 2 Monaten ist der Flascheninhalt gesintert und gelb verfärbt. Bei einer Lagerungstemperatur von 55°C ist das trocken abgefüllte Ifosfamid bereits innerhalb von 4 Tagen geschmolzen.

Demgegenüber ist beim erfindungsgemäß hergestellten Ifosfamid-Lyophilisat unter den vorgenannten Lagerbedingungen weder eine Verfärbung noch eine Veränderung der Konsistenz des Ifosfamids erkennbar.

Auch die Lösungsgeschwindigkeit des Ifosfamid-Lyophilisats ist gegenüber der Ifosfamid-Trockenabfüllung deutlich erhöht. Während sich das Lyophilisat unabhängig von der Lagerdauer bei der Zugabe des Lösungsmittels sofort löst, müssen die Injektionsflaschen mit der Trockenabfüllung nach Einspritzen des Lösungsmittels 1½ bis 3 Minuten kräftig geschüttelt werden. Wenn hierbei die Auflösung nicht sofort restlos erfolgt, und dies ist bei länger gelagerten Injektionsflaschen der Fall, ist es sogar erforderlich, die Lösung einige Minuten stehen zu lassen. Die Anwendung des Präparates in der Klinik wird dadurch erschwert.

Ifosfamid-Lyophilisat zeigt im Gegensatz zu Sterilkristallisat auch nach einer Lagerung von mehreren Jahren noch optimale Lösungseigenschaften. Außerdem ist die Ifosfamid-Trockenabfüllung (das heißt das reine Ifosfamid-Kristallisat) viel empfindlicher gegen Luftfeuchtigkeit als das Lyophilisat. So verflüssigt sich die Ifosfamid-Trockenabfüllung bereits bei einer relativen Luftfeuchtigkeit von unter 75%, während das Lyophilisat selbst bei 100% relativer Luftfeuchtigkeit zwar feucht wird, aber seine äußere Form behält.

Bei der Abfüllung des Sterilkristallisats ist ferner die Gefahr einer partikulären oder mikrobiellen Kontamination in wesentlich stärkerem Maße als beim Lyophilisat gegeben.

Bei der Herstellung des Ifosfamid-Lyophilisats erfolgt die Sterilfiltration der Lösung hingegen erst unmittelbar vor der Abfüllung in die Injektionsflaschen. Dadurch ist gegenüber der Abfüllung von Sterilkristallisat eine größere mikrobiologische Sicherheit gegeben. Auch partikuläre Verunreinigungen, die bei der Trockenabfüllung gelegentlich Anlaß zu Beanstandungen geben, lassen sich durch die Filtration der Lösung mit größerer Sicherheit vermeiden.

Die Lyophilisation des Ifosfamids führt jedoch nicht nur zu einer Produktverbesserung, sondern ist in den Herstellungskosten auch kostengünstiger als die Abfüllung von Sterilkristallisat.

Es hat sich gezeigt, daß nur das erfindungsgemäße Verfahren unter Verwendung eines Hexits, wie zum Beispiel Mannit, ein verbessertes Ifosfamid-Lyophilisat ergibt. Beispielsweise konnte durch Beimischung von Kochsalz, wie sie bei Trockenabfüllungen von anderen Oxazaphosphorinen üblich ist, kein Lyophilisat erhalten werden.

In der US-Patentschrift 4 537 883 wird zwar die Lyophilisierung einer ähnlichen Verbindung, und zwar des Cyclophosphamids in Gegenwart von Mannit beschrieben, jedoch ist es bei dem Lyophilisat gemäß diesem US-Patent wesentlich, daß dieses Lyophilisat einen bestimmten Anteil Wasser enthält (so lautet der Anspruch 1 des US-Patents 4 537 883 "hydrated lyophilisate"). Die Lyophilisierung gemäß diesem US-Patent erfolgt daher so, daß entweder ein gewisser Wasseranteil in dem Cyclophosphamid verbleibt, oder aber daß nach der Lyophilisierung ein gewisser Wasseranteil wieder zugeführt wird.

Die bessere Stabilität und Lagerfähigkeit des erfindungsgemäßen Lyophilisats ist demgegenüber überraschend.

Erfindungsgemäß wird beispielsweise eine wäßrige Lösung von Ifosfamid, die 1 bis 13 Gewichtsprozent an Ifosfamid enthält, sowie 0,1 bis 17 Gewichtsteile Hexit, bezogen auf einen Gewichtsteil Ifosfamid, gefriergetrocknet. Vorzugsweise enthält diese wäßrige Lösung 5 bis 12 Gewichtsprozent, insbesondere 8 bis 10 Gewichtsprozent Ifosfamid.

Es können auch entsprechende Ethanol-Wasser-Lösungen von Ifosfamid anstelle einer einen wässrigen Lösung verwendet werden (Ethanolanteil einer solchen Lösung bis zu 45% Gewicht in Gewicht, beispielsweise 1–20% Ethanol). In solchen Fällen wird möglichst zuerst das Ethanol vorzeitig im Vakuum entfernt, bevor das restliche Eis sublimiert wird, Die Bedingungen für die zuerst erfolgte Ethanolentfernung sind zum Beispiel: Druck $5 \times 10^{-1}$ mbar, Temperatur von −25°C auf −5°C steigend innerhalb von 10 Stunden, anschließend wird auf +22°C erhöht. Im einzelnen hängen diese Bedingungen auch von den unterschiedlichen Schichthöhen des zu trocknenden Gutes in den Injektionsflaschen ab und sind entsprechend zu variieren.

Die Menge an Hexit in dieser wäßrigen beziehungsweise wäßrig-ethanolischen Lösung beträgt im allgemeinen 1 bis 17, vorzugsweise 3 bis 12, insbesondere 5 bis 9 Gewichtsprozent. Bezieht man die Hexit-Menge auf einen Gewichtsteil Ifosfamid, dann ist die Hexit-Menge 0,1 is 17, vorzugsweise 0,1 bis 2,5, insbesondere 0,6 bis 0,8 Gewichtsteile Hexit pro 1 Gewichtsteil Ifosfamid. Als Hexit kommt in Frage: Mannit, Glucit, Sorbit, wie D-Sorbit, Dulcit, Allit, Altrit (zum Beispiel D- und L-Altrit), Idit (zum Beispiel D- und L-Idit), deren optisch aktive Formen (D- beziehungsweise L-Formen) sowie die entsprechenden Racemate. Insbesondere wird Mannit, wie D-Mannit, L-Mannit, DL-Mannit, Sorbit und/oder Dulcit verwendet, und zwar hiervon vorzugsweise D-Mannit. Als Hexit können auch Mischungen der genannten Hexite verwendet werden, zum Beispiel Mischungen von Mannit und Sorbit und/oder Dulcit. Da Dulcit weniger wasserlöslich ist als beispielsweise Mannit, soll der Dulcitgehalt in der wäßrigen Lösung beispielsweise 3 Gewichtsprozent nicht überschreiten. Mannit und Sorbit hingegen können beispielsweise in allen Verhältnissen gemischt werden.

Neben dem Hexit können auch noch andere, übliche pharmazeutische Hilfsstoffe zugefügt werden, wie zum Beispiel Glycin, Lactose, Polyvinylpyrrolidon, Glukose, Fructose, Albumin und äquivalente gerüstbildende Stoffe. Die Gesamtmenge an solchen Stoffen in der Lösung, die für die Gefriertrocknung eingesetzt wird, ist beispielsweise 0–16,9 Gewichtsteile, beispielsweise 0,1 bis 7 Gewichtsteile, bezogen auf 1 Gewichtsteil Ifosfamid. In dem fertigen Lyophilisat kann die Gesamtmenge an solchen Hilfsstoffen bis zu 16,9 Gewichtsteile, bezogen auf einen Gewichtsteil Hexit, betragen. Im einzelnen richtet sich die Menge an solchen Hilfsstoffen nach der vorhandenen Menge Hexit und zwar derart, daß die Gesamtmenge an Hexit und solchen anderen Hilfsstoffen in dem fertien Lyophilisat maximal nicht mehr als 17 Gewichtsteile beträgt, bezogen auf 1 Gewichtsteil Ifosfamid. Falls in dem Lyophilisat nur 0,1 Gewichtsteile an anderen Hilfsstoffen vorliegen; falls beispielsweise 8,5 Gewichtsteile Hexit vorliegen, kann zum Beispiel die Menge an anderen Hilfsstoffen bis zu 8,5 Gewichtsteile, bezogen auf 1 Gewichtsteil Ifosfamid, betragen.

Zur Herstellung der für die Gefriertrocknung einzusetzenden Lösung werden etwa 70 bis 83%, vorzugsweise 80% der erforderlichen Wassermenge beziehungsweise ethanolischer Wassermenge vorgelegt und die entsprechende Menge Ifosfamid und Mannit nacheinander (das heißt erst wird das Ifosfamid und anschließend der Mannit) unter ständigem Rühren beziehungsweise unter ständiger Bewegung gelöst. Nach vollständiger Auflösung wird das Endvolumen aufgefüllt und der pH-Wert gemessen. Der pH-Wert dieser Lösung soll beispielsweise nach dem Verdünnen zwischen 4 und 7 liegen. Vorzugsweise wird eine 4%ige Ifosfamid-Lösung hergestellt.

Die so erhaltende Ifosfamid-Lösung wird dann durch Filtration über hierfür übliche, keimdichte Filter

sterilisiert, und dann in entsprechende Behälter für Injektionspräparate abgefüllt. Die Aufbewahrungszeit bis zur Abfüllung in die Injektionsbehälter soll einschließlich der Zeit der Lösungsherstellung eine Zeit von 3 bis 4 Stunden nicht überschreiten, sofern es sich um Raumtemperatur (18 bis 22°C) handelt. Falls die anschließende Gefriertrocknung noch nicht sofort möglich ist, kann eine solche Lösung, gegebenenfalls auch nach Abfüllung in die Injektionsbehälter, beispielsweise noch bis zu 36 Stunden bei niederen Temperaturen, beispielsweise zwischen –5 und +10°C, vorzugsweise +4 bis +6°C aufbewahrt werden, bevor die Gefriertrocknung beginnt.

Zur Durchführung des erfindungsgemäßen Verfahrens wird dann die so erhaltene wässrige Ifosfamid-Lösung in Behälter für Injektionspräparate, beispielsweise Ampullen oder andere Glasgefäße eingefüllt, und die Lösung gefriergetrocknet.

Zur Sterilisation werden übliche keimdichte Filter, beispielsweise übliche Bakterienfilter mit einer Porengröße von etwa 0,2 µm verwendet. Die verwendeten Glasgefäße beziehungsweise Ampullen werden vorher in üblicher Weise sterilisiert.

Der verwendete Hexit (vorzugsweise Mannit, insbesondere D-Mannit) soll den Anforderungen der Britischen Pharmacopoeia 1980 entsprechen.

Der eingesetzte Hexit soll möglichst pyrogenfrei sein (Pyrogene sind Fieber erzeugende Endotoxine, die von Bakterien gebildet werden). Dasselbe gilt für das verwendete Ifosfamid. Die Entfernung beziehungsweise Zerstörung der Pyrogene erfolgt auf übliche Weise (beispielsweise wird die Wirkstofflösung vor der Sterilfiltration mit Aktivkohle behandelt). Ebenfalls soll das verwendete Injektionswasser steril und pyrogenfrei sein, und den Anforderungen des Deutschen Arzneibuches, 9. Ausgabe 1986 entsprechen. Als Injektionsgefäße werden zweckmäßig solche aus Röhrenglas beziehungsweise Hüttenglas der III. hydrolytischen Klasse verwendet (beispielsweise 10 R, 30 R und 50 H) (siehe hierzu Deutsches Arzneibuch, 9. Ausgabe 1986, Seiten 161–164 und DIN-Normen 58366 Teil 1 und Teil 5).

Weiterhin sollen die Injektionsgefäße sowie die weiteren Hilfsstoffe, wie Gummistopfen und Bördelkappen, den Anforderungen der DIN-Normen 58366, Teil 2 und Teil 3 sowie 58367, Teil 1 entsprechen.

Die Lösungsmengen der Ifosfamid-Lösungen, die für die Lyophilisation vorgesehen sind, in den jeweiligen Behältern (Ampullen) oder sonstigen Behältern für Injektionspräparate liegen pro Behälter zum Beispiel zwischen 1 und 500, vorzugsweise 1 und 250, insbesondere 2 und 50 ml. Die Behälter sind jeweils so zu bemessen, daß das hierin enthaltene Lyophilisat später in einer größeren Menge Flüssigkeit aufgelöst werden kann. Sie sollen daher im allgemeinen ein Volumen besitzen, das ausreicht, um eine gebrauchsfertige Endlösung herzustellen, die etwa das 2 bis 5, vorzugsweise 2 bis 4, insbesondere 2 bis 2,5fache des Volumens der ursprünglich eingefüllten Lyophilisat-Lösung hat.

Wie bereits erwähnt, wird vorzugsweise jede Ampulle beziehungsweise jedes Glasgefäß mit einer Einzeldosis von Ifosfamid gefüllt, wobei die Ifosfamid-Menge pro Glasgefäß beispielsweise zwischen 100 mg bis 10 g, vorzugsweise 200 mg bis 5 g beträgt. Anschließend wir die Lösung in diesem Glasgefäß oder der Ampulle in herkömmlicher Weise gefriergetrocknet. Es ist jedoch auch möglich, größere Mengen Ifosfamid, das heißt ein entsprechend größeres Lösungsvolumen der Ifosfamid-Lösung in einem entsprechend größeren Gefäß zu lyophilisieren, und anschließend das erhaltene Lyophilisat in entsprechende kleinere Dosierungen zu unterteilen beziehungsweise abzupacken.

Die Lyophilisierung selbst wird so durchgeführt, daß die Ampullen oder Glasgefäße oder sonstigen Gefäße, welche die Ifosfamid-Hexit-Lösung enthalten, unmittelbar auf eine Stellplatte oder in Tabletts auf einer Stellplatte in eine Gefriertrocknungskammer eingestellt werden. Nach dem Verschließen der Kammer werden die Ampullen beziehungsweise Gefäße auf Temperaturen unter 0°C abgekühlt, beispielsweise auf Temperaturen zwischen –70°C bis 0°C, vorzugsweise –50°C bis –30°C, insbesondere –45°C bis –35°C. Sobald die Lösungen vollständig gefroren sind, wird die Gefriertrocknungskammer allmählich evakuiert und mit dem Trocknen begonnen. Hierbei wird zuerst das nicht-adsorptiv gebundene Lösungsmittel entfernt, und zwar bei Temperaturen zwischen –30 bis +40°C, vorzugsweise 0°C bis +30°C, insbesondere +20°C bis +30°C, wobei ein Druck zwischen $10^{-3}$ bis 6, vorzugsweise $10^{-2}$ bis 2, insbesondere $10^{-1}$ bis 1 mbar eingestellt wird. Bei den zuvor angegebenen Temperaturen beziehungsweise Temperaturbereichen handelt es sich um die Temperatur der Stellplatten. Der Prozess wird dabei so gesteuert, daß die über die Plattentemperatur zugeführte Wärme vollständig als Sublimationswärme verbraucht wird, und die Temperatur der gefrorenen Ifosfamid-haltigen Lösung stets unterhalb ihrer eutektischen Temperatur bleibt. Die jeweils gewünschte Temperatur der Stellplatten kann zum Beispiel durch Programmscheiben oder Computer programmiert werden. Die Dauer zur Entfernung dieses nicht-adsorptiv gebundenen Lösungsmittels ist von der Größe der einzelnen Behälter abhängig und liegt beispielsweise zwischen etwa 4 bis 40 Stunden bei einer Plattentemperatur von +25°C und einem Druck von 0,8 mbar. Beispielsweise wird in diesem Zusammenhang auf die in dem Beispiel angegebenen Zeiten verwiesen.

Die vollständige Entfernung des nicht-adsorptiv gebundenen Wassers zeigt sich wie folgt an: Nicht adsorptiv gebundenes Wasser liegt als Eis vor. Durch die sogenannte Druckanstiegsmessung wird festgestellt, ob derartiges Wasser noch im Lyophilisat vorhanden ist. Dazu wird ein Ventil zwischen Trockenkammer und Kondensatorraum, an dem auch die Vakuumpumpe angeschlossen ist, geschlossen. Vorhandenes Eis würde dann schnell sublimieren und einen Druckanstieg in der Trockenkammer herbeiführen. Bei der Druckanstiegsmessung darf der Druck in der Kammer nach 15 Minuten vom Ausgangswert, zum Beispiel 0,8 mbar, höchstens auf 1 mbar ansteigen. Ein stärkerer Anstieg würde bedeuten, daß die Haupttrocknung noch nicht abgeschlossen ist.

Das noch vorhandene, restliche adsorptiv gebundene Lösungsmittel wird dann durch eine Nachtrocknung entfernt. Diese beträgt beispielsweise 3 bis 12 Stunden bei einem Vakuum von $10^{-1}$ bis $10^{-4}$ mbar, insbesondere 3–4 Stunden bei einem Vakuum von $10^{-3}$ bis $10^{-4}$ mbar.

Der Lyophilisationsprozeß ist beendet, wenn die Restfeuchte (bestimmt nach K. Fischer) unter 1%, vorzugsweise unter 0,5% liegt.

Insbesondere erfolgt die Nachtrocknung zur Entfernung von adsorptiv gebundenem Wasser bei Temperaturen zwischen 0 bis 40, vorzugsweise 10 bis 35, insbesondere 20 bis 30°C und einem Druck zwischen $10^{-4}$ bis $10^{-1}$, vorzugsweise $10^{-3}$ bis $10^{-2}$, insbesondere $10^{-3}$ bis $5 \times 10^{-3}$ mbar, wobei diese Nachtrocknung beispielsweise 2 bis 36, vorzugsweise 6 bis 24, insbesondere 3 bis 12 Stunden in Anspruch nimmt.

Nach Beendigung der Gefriertrocknung werden die Gefäße verschlossen. Das erfindungsgemäße Verfahren wird in sämtlichen Stufen unter sterilen Bedingungen durchgeführt.

Der Verschluß der Injektionsflaschen erfolgt dann zum Beispiel nach Belüftung der Gefriertrocknungskammer auf Normaldruck durch Zufuhr von trockener steriler Luft oder Stickstoff mit besonderen Gefriertrocknungs-Gummistopfen, die zur Vermeidung von Abrieb und zwecks Verbesserung der Gleitfähigkeit silikonisiert sind.

**Beispiel:**

Zur Gefriertrocknung wird folgende Lösung eingesetzt:
Ifosfamid 100 mg
D-Mannit 70 mg
Injektionswasser ad 1 ml

Die Dichte dieser Lösung beträgt 1,0563 g/ml bei + 6°C und 1,0527 g/ml bei + 20°C.

Die anzusetzende Lösungsmenge richtet sich nach der jeweiligen Abfüll- und Gefriertrocknungs-Kapazität.

Herstellung der Lösung:

Es werden ca. 80% Injektionswassermenge vorgelegt, und die entsprechende Menge Ifosfamid und Mannit in dem Wasser nacheinander unter ständigem Rühren gelöst. Nach vollständiger Auflösung wird auf das Endvolumen aufgefüllt und der pH-Wert gemessen.

Die fertige Lösung wird durch Filtration über hierfür übliche keimdichte Filter sterilisiert (zum Beispiel Sartorius SM 11107 oder SM 11307, 0,2 µm Porenweite, Pall Filter NRP (Porenweite 0,2 µm) und unter Vermeidung partikulärer und bakterieller Kontamination bis zur Abfüllung aufbewahrt. Eine Lagerung bei Raumtemperatur (20–22°C) soll einschließlich der Zeit der Lösungsherstellung 3–4 Stunden nicht überschreiten. Bei nicht sofortiger anschließender Gefriertrocknung kann die Lösung noch etwa 36 Stunden bei + 4°C bis + 6°C aufbewahrt werden.

Zur Sterilfiltration können zusätzlich übliche Vorfilter (zum Beispiel Sartorius SM 13400 oder Pall LPA) zum Schutz der Sterilfilter eingesetzt werden.

Reinigung der Injektionsflaschen:

Die Injektionsflaschen werden mit demineralisiertem Wasser warm und kalt und Luft gespült. Sämtliche Reinigungsmedien werden durch Filtration von Schwebstoffen befreit.

Unter Vermeidung von Rekontamination durch Partikel aus der Luft werden die Flaschen mittels Heißluft getrocknet und sterilisiert (diskontinuierlich bei 180°C/2 Stunden).

Die Reinigung der Gummistopfen, mit denen die Injektionsflaschen verschlossen werden, erfolgt unter Verwendung von demineralisiertem Wasser und beispielsweise einem Reinigungsmittel, bestehend aus nichtionogenen Tensiden und Phosphorsäureestern in wässriger Lösung.

Die gereinigten Stopfen werden unter Verwendung von demineralisiertem Wasser oder filtriertem demineralisiertem Wasser faser- und flusenfrei gespült. Die so gereinigten Stopfen werden dann mittels Dampf sterilisiert.

Die so gereinigten und sterilisierten Injektionsflaschen werden nun aseptisch mit der Ifosfamid-Lösung gefüllt und mit dem Gummistopfen versehen.

Füllmengen:

| | Füllmenge | Anwendungsvolumen* |
|---|---|---|
| Ifosfamid 200 mg | 2 ml | 5 ml |
| 500 mg | 5 ml | 12,5 ml |
| 1 g | 10 ml | 25 ml |
| 2 g | 20 ml | 50 ml |
| 5 g | 50 ml | 125 ml |

* für die spätere Verdünnung des Lyophilisats

| Die Füllvolumina sollen folgende Grenzen nicht überschreiten: | | |
|---|---|---|
| Füllvolumen | Grenzwerte der Einzelfüllvolumina | Durchschnittsgrenzwerte des Füllvolumens |
| 2 ml | 1,9 – 2,1 ml | 1,95 – 2,05 |
| 5 ml | 4,8 – 5,2 ml | 4,9 – 5,1 ml |
| 10 ml | 9,7 – 10,3 ml | 9,85 – 10,15 ml |
| 20 ml | 19,4 – 20,6 ml | 19,7 – 20,3 ml |
| 50 ml | 48,5 – 51,5 ml | 49,25 – 50,75 ml |

Die Füllvolumina sind statistisch zu überwachen, wobei mindestens alle 30 Minuten das Füllvolumen je Füllstelle einmal gemessen werden soll.

Die abgefüllten Injektionsflaschen werden so schnell wie möglich auf –40°C eingefroren.

Die Bedingungen für die Gefriertrocknung sind für die einzelnen Größen der Injektionsflaschen unterschiedlich. Es gelten beispielsweise die folgenden Werte:

Dauer der Haupttrocknung bei einer Plattentemperatur von +25°C und 0,6 mbar:
ca. 6–8 Stunden für Gefäße mit 200 mg Ifosfamid
ca. 10–12 Stunden für Gefäße mit 500 mg Ifosfamid
ca. 10–14 Stunden für Gefäße mit 1000 mg Ifosfamid
ca. 20–28 Stunden für Gefäße mit 2000 mg Ifosfamid
ca. 34 Stunden für Gefäße mit 5000 mg Ifosfamid

Dauer der Nachtrocknung ca. 3–4 Stunden unter Vakuum von 5 x $10^{-4}$ mbar, bei einer Plattentemperatur von +25°C.

Die Restfeuchte (nach K. Fischer bestimmt) soll unter 0,5% liegen.

Nach Beendigung der Gefriertrocknung werden die Injektionsflaschen verschlossen.

Zur Sicherung der Gummistopfen werden Bördelkappen aufgesetzt und anrolliert. Die fertigen Injektionsflaschen werden auf mechanische Defekte (Sprünge, fehlerhafter Verschluß etc.) kontrolliert.

## Patentansprüche

1. Lyophilisiertes Präparat, bestehend aus Ifosfamid und 0,1 bis 17 Gewichtsteilen Hexit, bezogen auf einen Gewichtsteil Ifosfamid sowie gegebenenfalls anderen üblichen pharmazeutischen Hilfsstoffen.

2. Lyophilisiertes Präparat nach Anspruch 1, dadurch gekennzeichnet, daß es als Hexit Mannit enthält.

3. Verfahren zur Herstellung eines Ifosfamid-Lyophilisates nach Anspruch 1, dadurch gekennzeichnet, daß man eine wässrige oder wässrig-ethanolische Lösung von Ifosfamid, die 1 bis 13 Gewichtsprozent Ifosfamid und 0,1 bis 17 Gewichtsteile Hexit, bezogen auf einen Gewichtsteil Ifosfamid, sowie gegebenenfalls 0 bis 16,9 Gewichtsteile (bezogen auf 1 Gewichtsteil Ifosfamid) weitere pharmazeutische Hilfsstoffe enthält, zwischen –70°C und 0°C einfriert, und dem so erhaltenen Produkt im gefrorenen Zustand das Wasser entzieht.

4. Verfahren nach Anspruch 3, dadurch gekennzeichnet, daß zuerst das nicht adsorptiv gebundene Wasser bei einer Temperatur zwischen –30°C und +40°C und einem Druck zwischen $10^{-3}$ bis 10 mbar und anschließend adsorptiv gebundenes Wasser bei einer Temperatur zwischen 0°C und 40°C und einem Druck zwischen $10^{-4}$ bis $10^{-1}$ mbar entfernt wird.

5. Verfahren nach einem oder mehreren der vorangegangenen Ansprüche, dadurch gekennzeichnet, daß als Hexit Mannit verwendet wird.

6. Ifosfamid-Lyophilisat, erhalten nach einem oder mehreren der Ansprüche 3–5.

## Revendications

1. Préparation lyophilisée, se composant d'ifosfamide, de 0,1 à 17 parties en poids d'hexitol pour 1 partie en poids d'ifosfamide et éventuellement d'autres adjuvants pharmaceutiques usuels.

2. Préparation lyophilisée selon la revendication 1, caractérisée en ce qu'elle contient du mannitol en tant qu'hexitol.

3. Procédé de préparation d'un lyophilisat d'ifosfamide selon la revendication 1, caractérisé en ce qu'on congèle entre –70°C et 0°C une solution aqueuse ou hydro-éthanolique d'ifosfamide qui contient de 1 à 13% en poids d'ifosfamide, de 0,1 à 17 parties en poids d'hexitol et éventuellement de 0 à 16,9 parties en poids (pour 1 partie en poids d'ifosfamide) d'autres adjuvants pharmaceutiques usuels, et on extrait l'eau du produit ainsi obtenu à l'état congelé.

4. Procédé selon la revendication 3, caractérisé en ce que l'eau non fixée par adsorption est d'abord

éliminée à une température comprise entre –30°C et +40°C et sous une pression comprise entre $10^{-3}$ et 10 mbars, puis l'eau fixée par adsorption est éliminée à une température comprise entre 0°C et sous une pression comprise entre $10^{-4}$ et $10^{-1}$ mbar.

5. Procédé selon la revendication 3 ou 4, caractérisé en ce que du mannitol est utilisé comme hexitol.

6. Lyophilisat d'ifosfamide, obtenu par le procédé selon l'une quelconque des revendications 3 à 5.

**Claims**

1. A lyophilized preparation consisting of ifosfamide and 0.1 to 17 parts by weight hexitol per part by weight ifosfamide and, optionally, other standard pharmaceutical auxiliaries.

2. A lyophilized preparation as claimed in claim 1, characterized in that it contains mannitol as the hexitol.

3. A process for the preparation of the ifosfamide lyophilizate claimed in claim 1, characterized in that an aqueous or aqueous-ethanolic solution of ifosfamide containing 1 to 13% by weight ifosfamide and 0.1 to 17 parts by weight hexitol per part by weight ifosfamide and, optionally, 0 to 16.9 parts by weight (per part by weight ifosfamide) of other pharmaceutical auxiliaries is frozen between –70°C and 0°C and the water is removed from the product thus obtained in its frozen state.

4. A process as claimed in claim 3, characterized in that first the non-adsorptively bound water is removed at a temperature of –30°C to +40°C and under a pressure of $10^{-3}$ to 10 mbar and then the adsorptively bound water is removed at a temperature of 0°C to 40°C and under a pressure of $10^{-4}$ to $10^{-1}$ mbar.

5. A process as claimed in one or more of the preceding claims, characterized in that mannitol is used as the hexitol.

6. Ifosfamide lyophilizate obtained in accordance with one or more of the claims 3 to 5.